# EUROPEAN PATENT SPECIFICATION

(11) **EP 3 284 464 B1**
(45) Date of publication and mention of the grant of the patent: **13.08.2025**
(21) Application number: 16780345.1
(22) Date of filing: 18.04.2016
(51) Int. Cl.: A61K 31/352, A61K 31/343, A61P 1/18

(54) **COMPOSITION FOR PREVENTING AND TREATING PANCREATITIS CONTAINING NAPHTHOQUINONE-BASED COMPOUND AS ACTIVE INGREDIENT**
ZUSAMMENSETZUNG ZUR VORBEUGUNG UND BEHANDLUNG VON PANKREATITIS MIT EINER NAPHTHOCHINONBASIERTEN VERBINDUNG ALS WIRKSTOFF
COMPOSITION POUR LA PRÉVENTION ET LE TRAITEMENT DE LA PANCRÉATITE CONTENANT DES COMPOSÉS À BASE DE NAPHTHOQUINONE EN TANT QUE PRINCIPE ACTIF

(30) Priority: 17.04.2015 KR 20150054364
(43) Date of publication of application: 21.02.2018
(73) Proprietor: NADIANBIO Ltd., Iksan-si, Jeollabuk-do 54538 (KR)
(72) Inventor: SO, Hong Seob, lksan-si Jeollabuk-do 54569 (KR); KIM, Hyung-Jin, lksan-si Jeollabuk-do 54636 (KR); SHEN, Aihua, lksan-si Jeollabuk-do 54645 (KR)
(74) Representative: Dehns
(86) International application number: PCT/KR2016/003998
(87) International publication number: WO 2016/167622

(56) References cited:
- WO-A1-2005/082354
- WO-A2-2005/082359
- WO-A2-2009/084835
- WO-A2-2011/035018
- WO-A2-2011/035018
- JP-A- 2006 182 732
- US-A- 6 114 113
- US-A1- 2005 187 288
- US-A1- 2005 192 247
- MOON ET AL: "Anti-inflammatory effects of @b-lapachone in lipopolysaccharide-stimulated BV2 microglia", INTERNATIONAL HNMUNOPHARMACOLOGY, ELSEVIER, AMSTERDAM, NL, vol. 7, no. 4, 21 February 2007 (2007-02-21), pages 506 - 514, XP005900666, ISSN: 1567-5769, DOI: 10.1016/J.INTIMP.2006.12.006

## Description

### BACKGROUND OF THE INVENTION

### 1. Field of the Invention

The present invention relates to a pharmaceutical composition for the prevention and treatment of pancreatitis which comprises the naphthoquinone-based compound dunnione, a pharmaceutically acceptable salt, or a solvate thereof as an active ingredient for the prevention and improvement of pancreatitis comprising the same.

### 2. Description of the Related Art

The pancreas is a long and flat organ having the length of 13 cm and the weight of about 100 g. The pancreas is located deep in the upper part of the belly button, under the stomach in the retroperitoneum. The important role of pancreas is the following two: the exocrine function to secrete digestive enzymes necessary for the digestion and absorption of food, and the endocrine function involved in hormone secretion to regulate *in vivo* metabolism like insulin regulating sugar.

Pancreatitis is a disease developed by the inflammation in the pancreas, which is divided into acute pancreatitis and chronic pancreatitis. Pancreatic juice contains such digestive enzymes as amylase (hydrolyze carbohydrate), trypsin (hydrolyze protein), and lipase (hydrolyze fat). Pancreatitis is developed when autolysis of the pancreas is induced by the enzymes above because the pancreatic juice does not flow smoothly due to alcohol abuse and gallstones, etc. There are two kinds of pancreatitis in general, which are mild type pancreatitis accompanied by interstitial edema and peripancreatic fat necrosis around the pancreas; and severe type pancreatitis accompanied by broad peripancreatic and intrapancreatic fat necrosis, pancreatic parenchymal necrosis, and hemorrhage (Bank PA., Am. J. gastroenterol., 89, pp151-152, 1994.; Bradley EL., Arch. Surg., 128, pp586-590, 1993.; Kim, C. H., Korean Journal of Gastroenterology, 46, pp321-332, 2005).

Pancreatitis is not only developed by the biliary disease caused by alcohol abuse and gallstones but also by various other reasons including metabolic disorders, drugs, and abdominal damage, etc. Pancreatitis is an inflammatory disease causing damage in pancreatic acinar cells, extensive interstitial edema, hemorrhage, and migration of neutrophilic granulocytes to the site of injury. Approximately 20% of pancreatitis patients undergo a severe clinical course involving multiple organ failures and systemic complications such as pancreatic necrosis and injury, with a high mortality rate of approximately 30%. The exact pathophysiological mechanism of pancreatitis has not been disclosed but is believed to be an autolysis process caused by the early activation of protease precursors in the pancreas. That is, once a digestive enzyme is abnormally prematurely activated in pancreas acinar cells, the pancreatic acinus itself is digested and accordingly the inflammation occurs to cause the separation and death of the pancreatic tissues. It has recently been reported that the activated macrophages infiltrating into pancreas after injury of pancreas acinar cells induce the secretion of the proinflammatory cytokine interleukin-1β (IL-13) as a response to the tissue damage, suggesting that the macrophages play an important role in circulation of inflammatory cells, pancreatic edema, and actual pancreas destruction.

The progression of acute pancreatitis can be divided into three stages; which are local inflammatory response, systemic inflammatory response causing one or multiple organ failure, and inflammation by the migration of intestinal bacteria into the pancreas. The early pathophysiological mechanism of pancreatitis has not been disclosed, yet. However, it has recently been known that when macrophages move into the pancreas after injury of pancreas acinar cells and induce the secretion of cytokines (IL-1 (interleukin-1), IL-6, and TNF-α (tumor necrosis factor-α)) in response to the tissue damage. The said cytokines play an important role in inflammatory cell circulation, pancreatic edema, and actual pancreas destruction. In the serum of acute pancreatitis patients, the increase of cytokines is observed. This increase is significantly higher in the cases with complications such as pancreatic necrosis, systemic inflammatory response, multiple organ failure, etc.
Several experimental treatment methods have been proposed to alleviate the severity of pancreatitis and inhibit the development of complications in various organs. However, when these experimental treatment methods were applied to patients, the effect was not so great, so that there are no approved drugs so far in relation to the prevention and treatment of pancreatitis.

In the meantime, the naphthoquinone-based compounds are known as active ingredients in some pharmaceutical compositions. Among them, β-lapachone is obtained from the laphacho tree (Tabebuia avellanedae) growing in South America, and dunnione and alpha-dunnione are obtained from the leaves of Streptocarpus dunnii growing in South America. Such natural tricyclic naphthoquinone derivatives have long been used as an anticancer agent and for the treatment of Chagas disease, one of the most representative endemic diseases in South America, and the effect thereof has been proved to be excellent. Since the pharmacological activity of the above compounds as an anticancer agent was known to the western world, they have been drawing people's attention and thereafter as described in US Patent No. 5,969,263, these tricyclic naphthoquinone derivatives have been developed as various types of anticancer agents by many study groups.

WO 2011/035018 A2 provides a pharmaceutical composition which comprises genetically modified cells and β-lapachone for the treatment of acute and chronic pancreatitis.

WO 2005/082359 A2 teaches methods that utilize agents such as β-lapachone and derivatives thereof in the treatment of pancreatic cancer and pre-cancerous pancreatic cancer conditions.

JP 2006 182732 A relates to trypsin inhibitors extracted from Tabebuia avellanedae bark which can be used in the treatment of pancreatitis.

WO 2009/084835 A2 discloses the use of β-lapachone and derivatives thereof for use in the treatment of kidney diseases.
wo 2005/082354 A1 teaches that cancers and /or malignancies can be treated by administration of a G1/S phase drug such as β-lapachone combined with an S phase drug.

Moon et. al, International Immunopharmaco 7, pages 506-514 (2007) reported on the inflammatory effects of β-lapachone in LPS stimulated BV2 microglia.

Nevertheless, there is no report that dunnione is effective in treating pancreatitis.

In the course of our study, the inventors confirmed that the naphthoquinone-based compound dunnione could reduce the pancreatic weight/body weight ratio, which is increased by pancreatitis; reduce the increased activities of digestive enzymes (amylase and lipase); reduce the expressions of cytokines (IL-1β (interleukin-1β) and MCP-1); reduce or inhibit the inflammation, edema, and cell necrosis of the pancreatic tissues; and inhibit the lung damage caused by pancreatitis, leading to the completion of this invention by proving the naphthoquinone-based compound, the pharmaceutically acceptable salt, or the solvate of the same could be effectively used as a composition for the prevention and treatment of pancreatitis.

### SUMMARY OF THE INVENTION

It is an object of the present invention to provide a pharmaceutical composition for the prevention and treatment of pancreatitis which comprises the naphthoquinone-based compound dunnione, a pharmaceutically acceptable salt, or a solvate of the same as an active ingredient.

To achieve the above object, the present invention provides a pharmaceutical composition which comprises the compound represented by formula 1 below, the pharmaceutically acceptable salt, or the solvate thereof as an active ingredient, for use in preventing and treating pancreatitis:

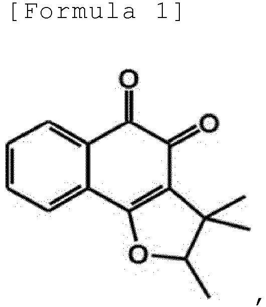

The present invention also provides a pharmaceutical composition which comprises the compound represented by formula 1, the pharmaceutically acceptable salt, or the solvate thereof as an active ingredient, for use in preventing and treating pancreatitis mediated lung damage.

### ADVANTAGEOUS EFFECT

The present invention relates to a composition comprising the naphthoquinone-based compound dunnione, a pharmaceutically acceptable salt, or a solvate of the same, for use in preventing and treating pancreatitis. Particularly, the naphthoquinone-based compound dunnione can reduce the pancreatic weight/body weight ratio, which is increased by pancreatitis; reduce the increased activities of digestive enzymes (amylase and lipase); reduce the expressions of cytokines (IL-1β (interleukin-1β) and MCP-1); reduce or inhibit the inflammation, edema, and cell necrosis of the pancreatic tissues; and inhibit the lung damage caused by pancreatitis. Therefore, the naphthoquinone-based compound dunnione, the pharmaceutically acceptable salt, or the solvate thereof can be effectively used as a composition for use in preventing and treating pancreatitis.

### BRIEF DESCRIPTION OF THE DRAWINGS

The application of the preferred embodiments of the present invention is best understood with reference to the accompanying drawings, wherein:
Figure 1 is a diagram illustrating the results of the measurement of the ratio of the pancreas weight to the body weight:
   ^{#}p<0.05: comparison between the normal control group and the caerulein treated group; and
   *p<0.05: comparison between the caerulein treated group and the dunnione treated group.
Figure 2 is a diagram illustrating the results of the measurement of the activities of amylase and lipase in serum:
   ^{#}p<0.05: comparison between the normal control group and the caerulein treated group; and
   *p<0.05: comparison between the caerulein treated group and the dunnione treated group.
Figure 3 is a diagram illustrating the results of the measurement of the concentration of IL-1β (interleukin-1β) in serum and the expression of IL-1β in pancreas:
   ^{#}p<0.05: comparison between the normal control group and the caerulein treated group; and
   *p<0.05: comparison between the caerulein treated group and the dunnione treated group.
Figure 4 is a diagram illustrating the results of the measurement of the expression of MCP-1 in pancreas:
   ^{#}p<0.05: comparison between the normal control group and the caerulein treated group; and
   *p<0.05: comparison between the caerulein treated group and the dunnione treated group.
Figure 5 is a set of a photograph showing the morphological changes of the pancreatic tissue in C57/BL6 mouse and a graph presenting the changes as histological scores:
   Cont: normal group;
   CAE: caerulein treated group;
   Dunnione: dunnione treated group;
   CAE+Dunnione 10: caerulein and dunnione treated group (10 mg/kg);
   CAE+Dunnione 20: caerulein and dunnione treated group (20 mg/kg);
   CAE+Dunnione 40: caerulein and dunnione treated group (40 mg/kg);
   ^{#}p<0.05: comparison between the normal control group and the caerulein treated group; and
   *p<0.05: comparison between the caerulein treated group and the dunnione treated group.

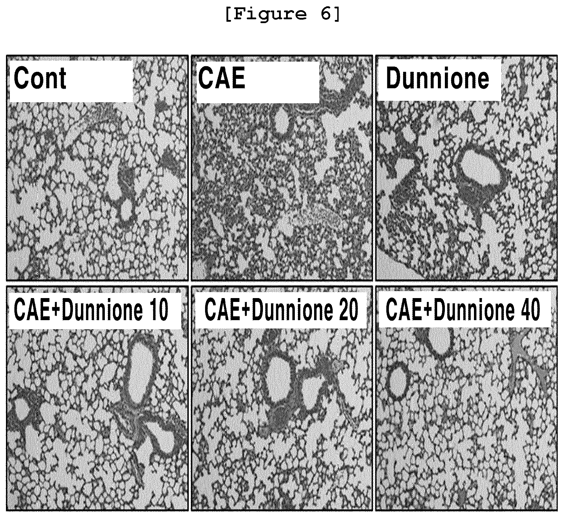

Figure 6 is a photograph illustrating the morphological changes of the lung tissue induced by dunnione in C57/BL6 mouse:
   Cont: normal group;
   CAE: caerulein treated group;
   Dunnione: dunnione treated group;
   CAE+Dunnione 10: caerulein and dunnione treated group (10 mg/kg);
   CAE+Dunnione 20: caerulein and dunnione treated group (20 mg/kg); and
   CAE+Dunnione 40: caerulein and dunnione treated group (40 mg/kg).

### DETAILED DESCRIPTION

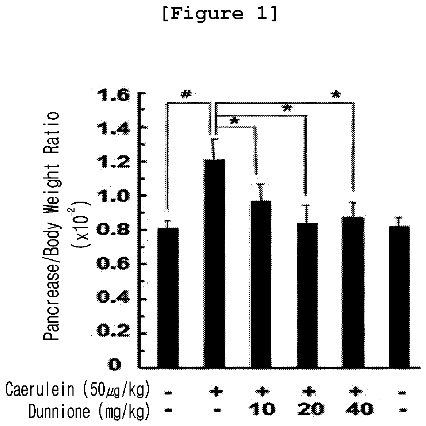

The present inventors induced pancreatitis in a mouse by administering caerulein (50 *µ*g/kg) intraperitoneally 6 times at 1 hour intervals. The mouse was orally administered with dunnione a day before the caerulein administration to prepare the pancreatitis animal model. The weight of pancreas was measured and the ratio of the pancreas weight by the body weight was calculated. As a result, while the ratio of pancreas weight / body weight of the caerulein treated group was significantly increased, compared with that of the control group, the ratio of pancreas weight / body weight of the dunnione treated group was significantly reduced dose-dependently (see Figure 1).

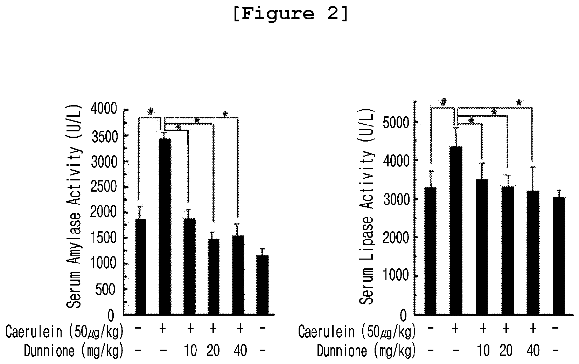

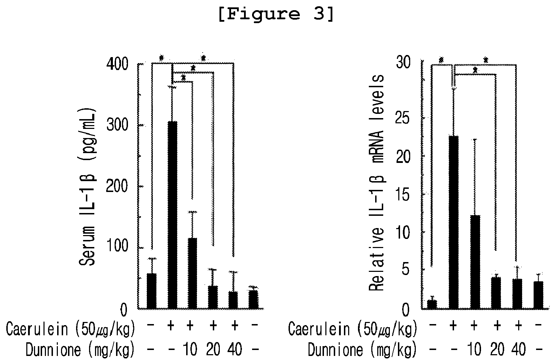

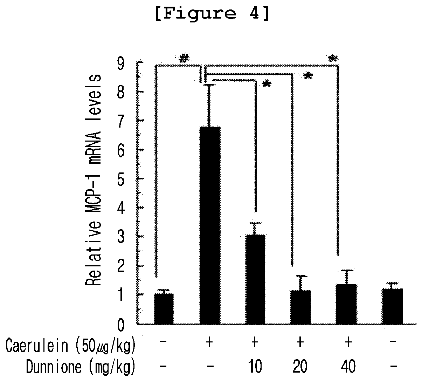

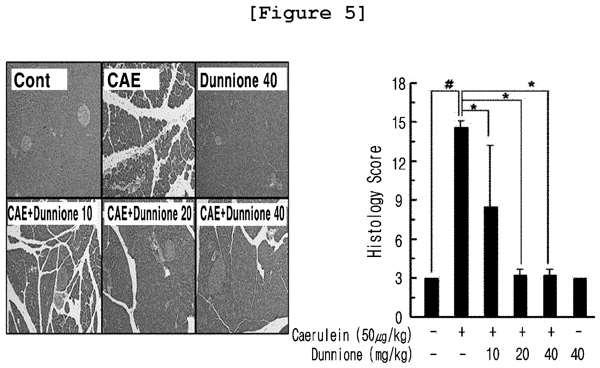

The present inventors investigated the activities of amylase and lipase, the digestive enzymes synthesized and secreted in pancreatic cells. As a result, the activity levels of both amylase and lipase in serum were higher in the caerulein treated group than in the normal control group, while the activities were significantly reduced in the dunnione treated group dose-dependently (see Figure 2). The expressions of IL-1β and MCP-1, the pancreatitis related cytokines, were also investigated. As a result, the expressions of IL-1β and MCP-1 and the concentration of IL-1β in serum were all very high in the caerulein treated group, while the expressions were reduced in the dunnione treated group dose-dependently (see Figures 3 and 4). The morphological change of the pancreatic tissue was also investigated. As a result, edema, inflammation, and necrosis were observed in the pancreatic tissue of the caerulein treated group. However, these phenomena were inhibited in the dunnione treated group dose-dependently (see Figure 5).

The present inventors also investigated the protective effect of dunnione, the naphthoquinone-based compound, on the lung damage caused by pancreatitis. As a result, the lung damage caused by caerulein was significantly inhibited by the cotreatment with dunnione in C57/BL6 mouse (see Figure 6). Therefore, the present inventors confirmed that the naphthoquinone-based compound dunnione can reduce the pancreatic weight/body weight ratio, which is increased by pancreatitis; reduce the increased activities of digestive enzymes; reduce the expressions of cytokines; reduce or inhibit the inflammation, edema, and cell necrosis in the pancreatic tissues; and inhibit the lung damage caused by pancreatitis. Therefore, the naphthoquinone-based compound dunnione, the pharmaceutically acceptable salt, or the solvate thereof can be effectively used as an active ingredient of a composition for the prevention and treatment of pancreatitis.

The naphthoquinone-based compound of the present invention, dunnione, can be used as a form of a pharmaceutically acceptable salt, in which the salt is preferably acid addition salt formed by pharmaceutically acceptable free acids. The acid addition salt herein can be obtained from inorganic acids such as hydrochloric acid, nitric acid, phosphoric acid, sulfuric acid, hydrobromic acid, hydriodic acid, nitrous acid, and phosphorous acid; non-toxic organic acids such as aliphatic mono/dicarboxylate, phenyl-substituted alkanoate, hydroxy alkanoate, alkandioate, aromatic acids, and aliphatic/aromatic sulfonic acids; or organic acids such as acetic acid, benzoic acid, citric acid, lactic acid, maleic acid, gluconic acid, methanesulfonic acid, 4-toluenesulfonic acid, tartaric acid, and fumaric acid. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate and mandelate.

The acid addition salt in this invention can be prepared by the conventional method known to those in the art. For example, the naphthoquinone-based compound of the present invention, dunnione, is dissolved in an organic solvent such as methanol, ethanol, acetone, methylenechloride, or acetonitrile, to which organic acid or inorganic acid is added to induce precipitation. Then, the precipitate is filtered and dried to give the salt. Or the same amount of the naphthoquinone-based compound dunnione and an acid or alcohol in water were heated, then the mixture was evaporated and dried or the precipitated salt was suction-filtered to give the same.

A pharmaceutically acceptable metal salt can be prepared by using a base. Alkali metal or alkali earth metal salt is obtained by the following processes: dissolving the compound in excessive alkali metal hydroxide or alkali earth metal hydroxide solution; filtering non-soluble compound salt; evaporating the remaining solution and drying thereof. At this time, the metal salt is preferably prepared in the pharmaceutically suitable form of sodium, potassium, or calcium salt. And the corresponding silver salt is prepared by the reaction of alkali metal or alkali earth metal salt with proper silver salt (ex; silver nitrate).

The present invention includes not only the naphthoquinone-based compound of the present invention, dunnione, but also a pharmaceutically acceptable salt thereof, and a solvate, or a hydrate produced from the same.

The addition salt in this invention can be prepared by the conventional method known to those in the art. For example, the naphthoquinone-based compound of the present invention, dunnione, is dissolved in water-miscible organic solvent such as acetone, methanol, ethanol, or acetonitrile, to which excessive organic acid or acid aqueous solution of inorganic acid is added to induce precipitation or crystallization. Then, the solvent or the excessive acid is evaporated from the mixture, followed by drying the mixture to give addition salt or suction-filtering the precipitated salt to give the same.

The pharmaceutical composition of the present invention comprising the naphthoquinone-based compound dunnione, the pharmaceutically acceptable salt, or the solvate of the same as an active ingredient can be administered orally or parenterally and be used in general forms of pharmaceutical formulation, but not always limited thereto.

The formulations for oral administration are exemplified by tablets, pills, hard/soft capsules, solutions, suspensions, emulsions, syrups, granules, elixirs, and troches, etc. These formulations can include diluents (for example, lactose, dextrose, sucrose, mannitol, sorbitol, cellulose, and/or glycine) and lubricants (for example, silica, talc, stearate and its magnesium or calcium salt, and/or polyethylene glycol) in addition to the active ingredient. Tablets can include binding agents such as magnesium aluminum silicate, starch paste, gelatin, methylcellulose, sodium carboxymethylcellulose and/or polyvinylpyrolidone, and if necessary disintegrating agents such as starch, agarose, alginic acid or its sodium salt or azeotropic mixtures and/or absorbents, coloring agents, flavours, and sweeteners can be additionally included thereto.

The pharmaceutical composition of the present invention comprising the naphthoquinone-based compound dunnione, the pharmaceutically acceptable salt, or the solvate of the same as an active ingredient can be administered by parenterally and the parenteral administration includes subcutaneous injection, intravenous injection, intramuscular injection and intrathoracic injection. To prepare the composition as a formulation for parenteral administration, the naphthoquinone-based compound dunnione, the pharmaceutically acceptable salt, or the solvate of the same of the present invention are mixed with a stabilizer or a buffering agent to produce a solution or suspension, which is then formulated as ampoules or vials. The composition herein can be sterilized and additionally contains preservatives, stabilizers, wettable powders or emulsifiers, salts and/or buffers for the regulation of osmotic pressure, and other therapeutically useful materials, and the composition can be formulated by the conventional mixing, granulating or coating method.

The effective dosage of the composition for use in the present invention can be adjusted according to the age, weight, and gender of patient, administration pathway, health condition, severity of disease, etc. For example, the effective dosage is 0.001 ~ 1,000 mg/day, and preferably 0.01 ~ 500 mg/day based on adult patients weighing 60 kg, which can be administered 1 ~ several times a day or the dosage can be divided and administered several times a day at a regular interval according to the judgment of a doctor or a pharmacist.

The pharmaceutical composition may contain contains the naphthoquinone-based compound dunnione at the concentration of 0.01 ~ 100 weight%, which can be varied with the form of medicine.

The present invention also provides a pharmaceutical composition for use in the prevention and treatment of pancreatitis mediated disease which comprises the compound represented by formula 1, the pharmaceutically acceptable salt, or the solvate thereof as an active ingredient.

The pancreatitis mediated disease herein is lung damage.

The naphthoquinone-based compound , dunnione, was confirmed to reduce the pancreatic weight/body weight ratio, which is increased by pancreatitis; reduce the increased activities of digestive enzymes; reduce the expressions of cytokines; reduce or inhibit the inflammation, edema, and cell necrosis of the pancreatic tissues; and inhibit the lung damage caused by pancreatitis. Therefore, the naphthoquinone-based compound, the pharmaceutically acceptable salt, or the solvate thereof can be effectively used as an active ingredient of a composition for the prevention and treatment of pancreatitis.

To be used for the prevention and improvement of pancreatitis as mentioned above, the naphthoquinone-based compound dunnione, the pharmaceutically acceptable salt, or the solvate thereof of the present invention can be prepared by various methods informed to those in the field of sitology or pharmacology. It can be prepared as it is or processed into any food form that can be taken orally by mixing them with a sitologically acceptable carrier, an excipient, or a diluent. Preferably, it can be prepared in the form of beverages, pills, granules, tablets, or capsules.

Practical and presently preferred embodiments of the present invention are illustrative as shown in the following Examples. Example 1 does not form part of the present invention.

### Example 1: Synthesis of β-lapachone

β-lapachone is obtained in a relatively small amount from lapacho trees, but lapachol, which is a raw material of β-lapachone synthesis, is obtained in a relatively large amount from lapacho trees. So, a method to synthesize β-lapachone by using lapachone was developed a long time ago. According to the method, β-lapachone can be obtained with a relatively good yield by mixing lapachol and sulfuric acid and stirring the mixture vigorously at room temperature.

In this example, in order to synthesize lapachol, 2-hydroxy-1,4-naphthoquinone (17.4 g, 0.10 M) was dissolved in DMSO (120 mℓ), to which LiH (0.88 g, 0.11 M) was slowly added. At this time, hydrogen could be generated, which needed to be carefully watched. During stirring the reaction solution, when the generation of hydrogen was not observed any more, the mixture was stirred further for 30 minutes, to which prenyl bromide (1-Bromo-3-methyl-2-butene, 15.9 g, 0.10 M) and LiI (3.35 g, 0.025 M) were slowly added. Then, the reaction solution was heated at 45°C and then stirred vigorously for 12 hours. The reaction solution was cooled down at 10°C or under, to which ice (76 g) was added and then water (250 mℓ) was added stepwise. The reaction solution was kept acidic (pH 1) by slowly adding concentrated hydrochloric acid (25 mℓ) . EtOAc (200 mℓ) was added to the reaction mixture, followed by stirring vigorously. As a result, a white solid that was not dissolved in EtOAc was generated. The obtained white solid was filtered and EtOAc layer was separated. Water layer was extracted by using EtOAc (100 mℓ) again, which was mixed with the organic layer extracted earlier. The organic layer was washed with 5% NaHCO₃ (150 mℓ), followed by concentration. The concentrate was dissolved in CH₂Cl₂ (200 mℓ), followed by separation using 2N NaOH (70 mℓ) with vigorous stirring. CH₂Cl₂ layer was separated twice by using 2 N NaOH aqueous solution (70 mℓ × 2). The separated solutions were combined together and pH of the mixed solution was adjusted to pH 2 by using concentrated HCl. Then, a solid was generated therein, which was separated by filtering. As a result, lapachol was obtained. The obtained lapachol proceeded to recrystallization by using 75% EtOH. The mixture was vigorously stirred with sulfuric acid (80 mℓ) at room temperature for 10 minutes, to which ice (200 g) was loaded to terminate the reaction.

The above process can be expressed with the following formula.

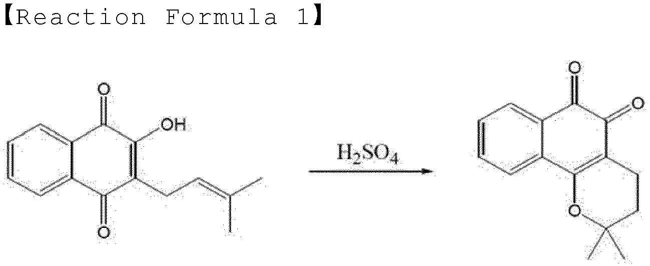

Next, CH₂Cl₂ (60 mℓ) was added thereto, and then the mixture was vigorously stirred. CH₂Cl₂ layer was separated and washed with 5% NaHCO₃. Water layer was extracted with CH₂Cl₂ (30 mℓ) once again and then washed with 5% NaHCO₃. The extracted water layer was mixed with the organic layer extracted earlier. The organic layer was dried over MgSO₄ and then concentrated, resulting in the obtainment of impure β-lapachone. The obtained impure β-lapachone was recrystallized by using isopropanol. As a result, pure β-lapachone (8.37 g) was obtained.

1H-NMR (CDCl3, δ): 8.05 (1H, dd, J=1, 8Hz), 7.82 (1H, dd, J=1, 8 Hz), 7.64 (1H, dt, J=1, 8 Hz), 7.50 (1H, dt, J=1, 8 Hz), 2.57 (2H, t, J=6.5 Hz), 1.86 (2H, t, J=6.5 Hz) 1.47 (6H, s).

### Example 2: Synthesis of dunnione

The solid not dissolved in EtOAc, which was separated during the production of lapachol in Example 1 was O-alkylated 2-prenyloxy-1,4-maphthoquinone, which was a different material from the C-alkylated lapachol. The obtained O-alkylated 2-prenyloxy-1,4-maphthoquinone was recrystallized by using EtOAc once again for the purification. The purified solid (3.65 g, 0.015 M) was dissolved in toluene, followed by reflux for 5 hours to induce claisen rearrangement. The toluene was concentrated by distillation under reduced pressure, which was mixed with sulfuric acid (15 mℓ) without any further purification process, followed by vigorous stirring at room temperature for 10 minutes. Ice (100 g) was added thereto to terminate the reaction. CH₂Cl₂ (50 mℓ) was added thereto as a reactant, and then the mixture was stirred vigorously. CH₂Cl₂ layer was separated and washed with 5% NaHCO₃. Water layer was extracted with CH₂Cl₂ (20 mℓ) once again and then washed with 5% NaHCO₃. The extracted water layer was mixed with the organic layer extracted earlier. The organic layer was dried over MgSO₄ and then concentrated, followed by silica gel chromatography. As a result, pure dunnione (2.32 g) was obtained.

1H-NMR (CDCl3, δ): 8.05 (1H, d, J=8Hz), 7.64 (2H, d, J=8Hz), 7.56 (1H, m), 4.67 (1H, q, J=7Hz), 1.47 (3H, d, J=7Hz), 1.45(3H, s) 1.27 (3H, s).

### Example 3: Synthesis of α-dunnione

2-Prenyloxy-1,4-maphthoquinone (4.8 g, 0.020 M) purified in Example 2 was dissolved in xylene, followed by reflux for 15 hours. Claisen rearrangement was induced at a higher temperature for a longer time than those of Example 1. In this process, α-dunnione in which claisen rearrangement and cyclization reaction with the lapachol derivative wherein one of two methyl groups has migrated were accomplished was obtained. Then, xylene was concentrated by distillation under reduced pressure, followed by silica gel chromatography. As a result, pure α-dunnione (1.65 g) was obtained.

1H-NMR (CDCl3, δ): 8.06 (1H, d, J=8Hz), 7.64 (2H, m), 7.57 (1H, m), 3.21 (1H, q, J=7Hz), 1.53 (3H, s), 1.51(3H, s) 1.28 (3H, d, J=7Hz).

### Experimental Example 1: Preparation of pancreatitis animal model

In this invention, C57BL/6 mice at the body weight of about 22±2 g were used. All the mice were raised in a sterile animal laboratory with constant temperature (22~26°C) and humidity (55~60%). The mice were adapted for 1 week while feeding with normal solid feed (Samtaco Korea) and water. All the experiments were performed according to the guide for the care and use of laboratory animals set by Wonkwang University. Among the many methods for inducing pancreatitis, the caerulein mediated pancreatitis model is the most common animal model due to the excellent reproducibility and economic feasibility, and accordingly this animal model has been well studied relatively by many research groups particularly about the development mechanism of the disease. All the experimental animals stopped being supplied with feeds from 16 hours before the experiment except the normal group, and from that moment caerulein (50 *µ*g/kg) was intraperitoneally administered 6 times at one hour interval to induce pancreatitis. One day before the caerulein administration, dunnione was orally administered in order to investigate the preventive effect of dunnione on caerulein mediated pancreatitis.

### Experimental Example 2: Decrease of pancreas weight by dunnione

When pancreatitis is developed, the weight of pancreas is known to be increased because of edema. Thus, blood was extracted from all the animals finished with the experiment and then the ratio of the pancreas weight to the body weight was measured.

Particularly, the animal model prepared by the same manner as described in Experimental Example 1 was treated with dunnione at different concentrations (0, 10, 20, 40 mg/kg), to which caerulein (50 *µ*g/kg) was administered to induce pancreatitis. After blood was extracted, the pancreas was cut out and the weight of the pancreas to the body weight was measured by the pancreas weight (wet pancreas weight) /body weight (wet body weight) formula of Grady method.

As a result, as shown in Figure 1, the ratio of pancreas weight/body weight was significantly increased in the caerulein treated group. In the meantime, in the group treated with dunnione, the ratio of pancreas weight/body weight was significantly reduced dose-dependently (Figure 1).

### Experimental Example 3: Inhibitory effect of dunnione on digestive enzyme activity

Pancreas is the organ that synthesizes protein most actively, so that it synthesizes and secrets 6-20 mg of digestive proteins a day. These digestive enzymes have a strong proteolytic activity, so that various safety systems are working in pancreatic acinar cells to prevent self-digestion of pancreas. Pancreatitis occurs by the self-digestion of pancreas induced when the digestive enzymes normally synthesized and secreted in pancreas cells as inactive precursors is abnormally early activated. Thus, the present inventors investigated the activities of amylase and lipase in serum.

Particularly, the animal model prepared by the same manner as described in Experimental Example 1 was treated with dunnione by the same manner as described in Experimental Example 2, followed by caerulein administration. Serum was obtained and then OD₅₉₅ and OD₄₁₂ were measured by using amylase activity analysis kit (DAMY-100; BioAssay Systems, USA) and lipase activity analysis kit (DLPS-100; BioAssay Systems, USA) to investigate the activities of amylase and lipase.

As a result, as shown in Figure 2, the amylase activity was significantly increased in the caerulein treated group (1700±106.4 U/L), compared with the normal group (1168±215.0 U/L). The lipase activity was also higher in the caerulein treated group about 30% by the normal group. In the dunnione treated group, the amylase activity and the lipase activity were significantly reduced dose-dependently (Figure 2).

### Experimental example 4: Inhibitory effect of dunnione on the expressions of inflammation mediators

### <4-1> Inhibitory effect of dunnione on the expression of IL-1β

Pancreatitis is caused by the damage of pancreas cells by self-activation of digestive enzymes in pancreatic acinar cells. When various immune cells are activated to cause inflammation, the damage of pancreas is enlarged. Then, various humoral factors including inflammatory cytokines produced from the immune cells are involved in the inducement of systemic organ damage. The most representative factors involved in the activation of immune cells causing inflammatory reaction in pancreatitis are TNF-α, interleukin-1β (IL-1β), and interleukin-6. Particularly, serum IL-1β and TNF-α are up-regulated in the case of pancreatitis. According to the previous reports, they are also closely related to the severity of pancreatitis. The experiment using the interleukin 1 knock-out mouse proved that when interleukin 1 receptor antagonist was treated to the animal, the development of pancreatitis was inhibited, indicating that IL-1β was a major factor to cause pancreatitis. Therefore, any change in the expression of IL-1β was investigated.

Particularly, the animal model prepared by the same manner as described in Experimental Example 1 was treated with dunnione by the same manner as described in Experimental Example 2, followed by caerulein administration. Serum was obtained from the animal model and then the level of serum IL-1β was measured by using mouse IL-1β ELISA kit (BD, USA). First, nasal lavage fluid or diluted serum was loaded in the 96-well plate coated with the mouse IL-1β specific antibody, followed by reaction at room temperature for 2 hours. After washing the plate, biotin conjugate was added thereto, followed by reaction at room temperature for 1 hour. After washing the plate, streptavidin-HRP working solution was added thereto, followed by reaction. After washing the plate again, stabilized chromogen was added thereto, followed by reaction. Then, OD₄₅₀ was measured. In addition, the expression of IL-1β in pancreas was confirmed by real-time PCR in each pancreas tissue. To extract total RNA, 1 mL of TRIzol (Invitrogen, USA) was added to 20 mg of each pancreas tissue, which stood on ice for 5 minutes to lyse cells. 200 µL of chloroform was added thereto, followed by centrifugation at 14,000 rpm for 15 minutes. The supernatant was obtained and transferred into a new tube, to which equal amount of isopropanol was added, followed by centrifugation at 14,000 rpm for 10 minutes to separate RNA. 99% ethanol was added thereto, followed by centrifugation at 2,000 rpm. After washing once again, the RNA pellet was dried in air and then dissolved in diethylpyrocarbonate (DEPC) water. The RNA was quantified by using Nanodrop 2000 (Thermo, USA). 2 µg of total RNA was heated with DEPC together at 70°C for 5 minutes, which was added to Reverse Transcription Premix (Invitrogen, USA) containing oligo (dT), whose final volume was adjusted to be 20 µL. The reaction mixture was reacted at 42°C for 55 minutes and at 70°C for 15 minutes to synthesize cDNA. The synthesized cDNA was used for polymerase chain reaction (PCR). To amplify IL-1β and GAPDH from the obtained cDNA, PCR was performed with 2 µL of the cDNA diluted 5 times in DEPC, 0.5 µL of primer, 7 µL of DEPC water, and 10 µL of SYBR green Master Mix (Invitrogen Life Technology, USA) using StepOne Plus Real-Time PCR system (Applied Biosystems, USA). Reaction condition was as follows: 50°C for 2 minutes, 95°C for 10 minutes, 95°C for 10 seconds and 60°C for 1 minute, and this cycle was repeated 40 times. Primer sequences of the genes to be amplified are shown in Table 1 below.

**[Table 1]**

| Gene | Primer | Nucleotide Sequence | Directi on | SEQ. ID. NO: |
|---|---|---|---|---|
| IL-1β | IL-1b_F | TCT TTG AAG TTG ACG GAC CC | Forward | 1 |
| | IL-1b_R | TGA GTG ATA CTG CCT GCC TG | Reverse | 2 |
| GAPDH | GAPDH_F | TCC CAC TCT TCC ACC TTC GA | Forward | 3 |
| | GAPDH_R | AGT TGG GAT AGG GCC TCT CTT G | Reverse | 4 |

As a result, as shown in Figure 3, the serum IL-1β level was significantly high in the caerulein treated group, compared with the normal group. In the meantime, the serum IL-1β level was significantly reduced in the group treated with dunnione dose-dependently. The expression of IL-1β in pancreas was significantly high in the caerulein treated group, compared with the normal group, while the expression of IL-1β in pancreas was significantly reduced in the group treated dunnione (Figure 3).

### <4-2> Inhibitory effect of dunnione on the expression of MCP-1

For the infiltration of inflammatory cells, a series of processes such as activation of inflammatory cells and migration of inflammatory cells from blood vessels into tissues are required. Chemokine is a kind of cytokine that helps inflammatory cells to move to tissue sites with its activity such as chemokinesis and chemotaxis. In particular, in the case of pancreatitis, MCP-1 is known to be involved in the developments of pancreatitis and lung damage, the pancreatitis complication. Thus, the present inventors investigated the MCP-1 expression pattern.

Particularly, the animal model prepared by the same manner as described in Experimental Example 1 was treated with dunnione and caerulein by the same manner as described in Experimental Example 2. RT-PCR was performed with each pancreas tissue by the same manner as described in Example <4-1> to investigate the expression pattern of MCP-1. Primer sequences of the genes to be amplified are shown in Table 2 below.

**[Table 2]**

| Gene | Primer | Nucleotide Sequence | Directi on | SEQ. ID. NO: |
|---|---|---|---|---|
| MCP-1 | MCP-1_F | GGT CCC TGT CAT GCT TCT GG | Forward | 5 |
| | MCP-1_R | CCT TCT TGG GGT CAG CAC AG | Reverse | 6 |
| GAPDH | GAPDH_F | TCC CAC TCT TCC ACC TTC GA | Forward | 3 |
| | GAPDH_R | AGT TGG GAT AGG GCC TCT CTT G | Reverse | 4 |

As a result, as shown in Figure 4, the expression of MCP-1 was significantly high in the caerulein treated group, compared with the normal group. The expression of MCP-1 in pancreas in the group treated with dunnione was reduced dose-dependently (Figure 4).

### Experimental Example 5: Morphological changes of pancreas tissue induced by dunnione

The present inventors investigated the morphological changes of pancreas tissue based on the previous reports saying that inflammation, edema, and cell necrosis were found in pancreatitis.

Particularly, the animal model prepared by the same manner as described in Experimental Example 1 was treated with dunnione and caerulein by the same manner as described in Experimental Example 2. Pancreas tissues were separated, fixed in 10% formalin for 24 hours at 4°C, demineralized with 10% ethylenediaminetetra-acetic acid (EDTA, pH 7.4) solution, washed, dehydrated, embedded in paraffin and cut into 5 µm sections. The sections were stained with hematoxylin-eosin, followed by observation under optical microscope.

As a result, as shown in Figure 5, pancreatic acinar cells were arranged densely in the normal group, while the size of the pancreatic acinar cell was bigger and the distance between the cells was farther due to edema in the caerulein treated group. Infiltration and necrosis of the inflammatory cells were also observed in the caerulein treated group. However, these phenomena shown in the caerulein treated group were inhibited to be back to almost normal by the treatment of dunnione. As a result of graphing, it was confirmed that the edema, inflammation, and cell necrosis of pancreas tissues induced by caerulein were all inhibited by the treatment of dunnione dose-dependently (Figure 5).

### Experimental Example 6: Protective effect on lung damage induced by pancreatitis

### <6-1> Protective effect of dunnione on lung damage

The present inventors confirmed that the pancreatitis-induced lung damage could be suppressed by dunnione.

Particularly, C57/BL6 mouse prepared by the same manner as described in Experimental Example 1 was treated with dunnione and caerulein by the same manner as described in Experimental Example 2. Then, the pancreas tissues were stained with hematoxylin-eosin by the same manner as described in Experimental Example 5, followed by observation under optical microscope.

As a result, as shown in Figure 6, it was confirmed that lung damage induced by caerulein was significantly inhibited by the cotreatment of dunnione in C57/BL6 mouse (Figure 6).

Based on the results above, the naphthoquinone-based compound dunnione was confirmed to have an excellent protective effect on caerulein mediated pancreatitis and lung damage induced by pancreatitis by regulating various inflammatory responses.

## Claims

1. A pharmaceutical composition comprising the compound represented by formula 1 below, the pharmaceutically acceptable salt, or the solvate thereof as an active ingredient, for use in preventing or treating pancreatitis:

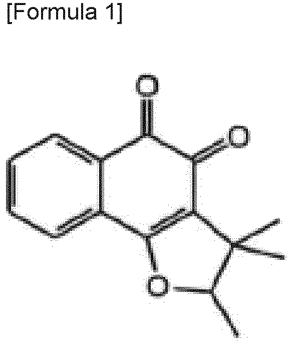

2. The pharmaceutical composition for use in preventing or treating pancreatitis of claim 1, wherein the pancreatitis is chronic pancreatitis or acute pancreatitis.

3. The pharmaceutical composition for use in preventing or treating pancreatitis of claim 1, wherein the composition characteristically reduces the activities of serum amylase and lipase.

4. The pharmaceutical composition for use in preventing or treating pancreatitis of claim 1, wherein the composition characteristically inhibits the expression of IL-1β or MCP-1.

5. A pharmaceutical composition comprising the compound represented by formula 1 below, the pharmaceutically acceptable salt, or the solvate thereof as an active ingredient, for use in preventing or treating pancreatitis mediated lung damage:

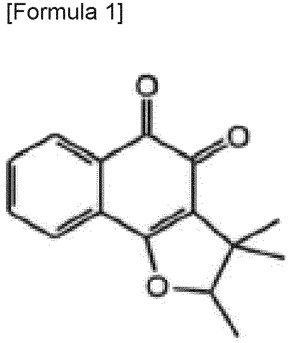

## Patentansprüche

1. Pharmazeutische Zusammensetzung, umfassend die Verbindung, die durch die nachstehende Formel 1 dargestellt ist, das pharmazeutisch verträgliche Salz oder das Solvat davon als Wirkstoff zur Verwendung bei der Vorbeugung oder Behandlung von Pankreatitis:

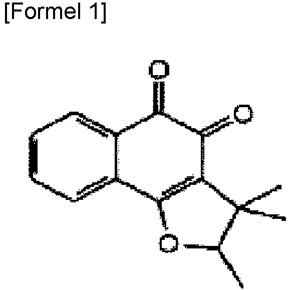

2. Pharmazeutische Zusammensetzung zur Verwendung bei der Vorbeugung oder Behandlung von Pankreatitis nach Anspruch 1, wobei die Pankreatitis eine chronische Pankreatitis oder eine akute Pankreatitis ist.

3. Pharmazeutische Zusammensetzung zur Verwendung bei der Vorbeugung oder Behandlung von Pankreatitis nach Anspruch 1, wobei die Zusammensetzung charakteristischerweise die Aktivitäten von Serumamylase und Lipase reduziert.

4. Pharmazeutische Zusammensetzung zur Verwendung bei der Vorbeugung oder Behandlung von Pankreatitis nach Anspruch 1, wobei die Zusammensetzung charakteristischerweise die Expression von IL-1β oder MCP-1 hemmt.

5. Pharmazeutische Zusammensetzung, umfassend die Verbindung, die durch die nachstehende Formel 1 dargestellt ist, das pharmazeutisch verträgliche Salz oder das Solvat davon als Wirkstoff zur Verwendung bei der Vorbeugung oder Behandlung von durch Pankreatitis verursachten Lungenschäden:

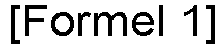

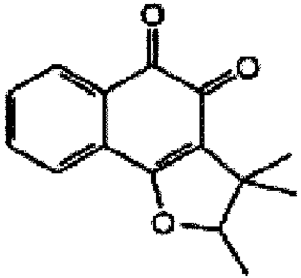

## Revendications

1. Composition pharmaceutique comprenant le composé représenté par la formule 1 ci-dessous, le sel pharmaceutiquement acceptable, ou le solvate de celui-ci en tant qu'ingrédient actif, pour utilisation dans la prévention ou le traitement de la pancréatite :

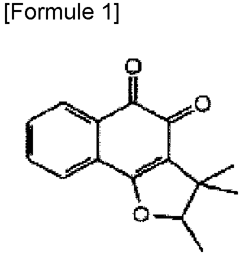

2. Composition pharmaceutique pour utilisation dans la prévention ou le traitement de la pancréatite selon la revendication 1, dans laquelle la pancréatite est une pancréatite chronique ou une pancréatite aiguë.

3. Composition pharmaceutique pour utilisation dans la prévention ou le traitement de la pancréatite selon la revendication 1, dans laquelle la composition réduit de manière caractéristique les activités de l'amylase et de la lipase sériques.

4. Composition pharmaceutique pour utilisation dans la prévention ou le traitement de la pancréatite selon la revendication 1, dans laquelle la composition inhibe de manière caractéristique l'expression de IL-1β ou MCP- 1.

5. Composition pharmaceutique comprenant le composé représenté par la formule 1 ci-dessous, le sel pharmaceutiquement acceptable, ou le solvate de celui-ci en tant qu'ingrédient actif, pour utilisation dans la prévention ou le traitement de lésions pulmonaires dues à la pancréatite :
